# EUROPEAN PATENT SPECIFICATION

(11) **EP 1 350 855 B1**
(45) Date of publication and mention of the grant of the patent: **26.10.2005**
(21) Application number: 03252054.6
(22) Date of filing: 31.03.2003
(51) Int. Cl.: C12Q 1/68

(54) **Array based hybridization assays**
Auf Array-basierende Hybridisierungstests
Tests d'hybridation reposant sur un système en rangées (array)

(30) Priority: 01.04.2002 US 114668
(43) Date of publication of application: 08.10.2003
(73) Proprietor: Agilent Technologies, Inc. - a Delaware corporation -, Palo Alto, CA 94303-0870 (US)
(72) Inventor: Ilsley, Diana D., San Jose, CA 95123 (US); Tsang, Peter, Daly City, CA 94015 (US); Caren, Michael P., Palo Alto, CA 94303 (US); Amorese, Douglas A., Los Altos, CA 94022 (US)
(74) Representative: Tollett, Ian

(56) References cited:
- WO-A-01/92569
- US-A- 5 861 251
- US-A- 6 153 412
- US-A1- 2001 036 632
- LINDROOS K ET AL: "Minisequencing on oligonucleotide microarrays: comparison of immobilisation chemistries." NUCLEIC ACIDS RESEARCH. ENGLAND 1 JUL 2001, vol. 29, no. 13, 1 July 2001 (2001-07-01), pages E69-E69, XP002251355 ISSN: 1362-4962
- PASTINEN T ET AL: "A SYSTEM FOR SPECIFIC, HIGH-THROUGPUT GENOTYPING BY ALLELE-SPECIFIC PRIMER EXTENSION ON MICROARRAYS" GENOME RESEARCH, COLD SPRING HARBOR LABORATORY PRESS, US, vol. 10, no. 7, July 2000 (2000-07), pages 1031-1042, XP008013561 ISSN: 1088-9051

## Description

The present invention relates to a method of assaying a sample for the presence of one or more nucleic acid analyte members of a nucleic acid set, and to an array.

The characterization of cellular gene expression (i.e., gene expression analysis) finds application in a variety of disciplines, such as in the analysis of differential expression between different tissue types, different stages of cellular growth or between normal and diseased states.

Fundamental to differential expression analysis is the detection of different mRNA species in a test sample, and often the quantitative determination of different mRNA levels in that test sample. In order to detect different mRNA levels in a given test population, a population of labeled target nucleic acids that, at least partially, reflects or mirrors the mRNA profile of the test sample, is first produced. In other words, a population of labeled target nucleic acids is generated where at least a portion of the mRNA species in the test sample are represented, in terms of presence and often in terms of amount. Following target generation, the target population is contacted with one or more probe sequences, e.g., as found on an array. Following contact, any resultant binding complexes of probe and target sequences are detected to obtain assay data which is then employed to determine the presence and often amount of specific targets in the target population. As such, from the resultant data, information about the mRNAs present in the sample, i.e., the mRNA profile and gene expression profile, can be readily deduced.

Because of the widespread applicability of the above-described methods, e.g., in differential gene expression analysis and related applications, there is continued interest in the development of improved methods of determining mRNA expression profiles in a sample. Of particular interest is the development of protocols that reduce different reagent addition steps and are adaptable to automated and/or high throughput formats.

U.S. Patents disclosing the use of inkjet devices to dispense bio/chemical agents such as proteins and nucleic acids include: U.S. Patent No. 4,877,745; 5,073,495; 5,200,051; 5,338,688; 5,474,796; 5,449,754; 5,658,802; 5,700,637; 5,751,839; 5,891,394; 5,958,342, 6,221,653, and 6,112,605. Also of interest is Roda et al., Biotechniques (2000) 28:492-496; Graves et al., Anal. Chem. (1998) 70: 5085-5092; and Yershov et al., Proc. Nat'l Acad. Sci. USA (1996) 93: 4913-4918.

US 2001/0036632 discloses methods for solid phase polymerase-mediated amplification using immobilized primers on a microarray for detecting and cloning multiple target polynucleotides. The methods are useful for research and clinical applications, such as large scale assays of genetic information in biological samples of interest.

US 6,153,412 discloses a lyophilized reagent for PCR which is prepared by adding a stabilizing and sedimenting agent to an aqueous reaction mixture and lyophilizing thereof. The lyophilized PCR reagent leads to a simplification of multistep PCR manipulation, an increase in heat stability of the reaction mixture, and reduction of carry-over contamination. The lyophilized PCR reagent can be used in analysis of DNA sequences and for diagnosis of diseases.

Methods for assaying a sample for the presence of one or more nucleic acid analytes, either qualitatively or quantitatively, are provided. In the subject methods, an array of DNA primer compositions is contacted with the sample being assayed under conditions sufficient to produce labeled target nucleic acids enzymatically on the surface of the array at locations where a DNA primer has hybridized to a nucleic acid analyte to produce a duplex nucleic acid. The presence of labeled target nucleic acids on the array surface is then detected and used to determine the presence of the one or more nucleic acid analytes in the sample. Also provided are kits for practicing the subject methods. The subject invention finds use in a variety of different applications, including differential gene expression analysis.

### DEFINITIONS

The term "nucleic acid" as used herein means a polymer composed of nucleotides, e.g. deoxyribonucleotides or ribonucleotides.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to up to about 100 nucleotides in length.

The term "polynucleotide" as used herein refers to a single or double stranded polymer composed of nucleotide monomers of generally greater than 100 nucleotides in length and up to about 8,000 or more nucleotides in length. Polynucleotides include single or multiple stranded configurations, where one or more of the strands may or may not be completely aligned with another.

A "nucleotide" refers to a subunit of a nucleic acid and includes a phosphate group, a 5 carbon sugar and a nitrogen containing base, as well as analogs of such subunits.

The term "cDNA" as used herein means a complementary DNA molecule made as a copy of mRNA amplified using PCR for deposition on arrays. cDNAs can range from about 100 bp to about 8,000 bp, where average cDNAs are typically 1 to 2 kb in length. cDNA can also refer to a complementary DNA molecule made as a copy of mRNA using reverse transcriptase and be a single or double stranded molecule.

The term "array" as used herein means a substrate having a plurality of binding agents stably attached to its surface, where the binding agents may be spatially located across the surface of the substrate in any of a number of different patterns.

Methods for assaying a sample for the presence of one or more nucleic acid analytes, either qualitatively or quantitatively, are provided. In the subject methods, an array of DNA primer compositions is contacted with the sample being assayed under conditions sufficient to produce labeled target nucleic acids enzymatically on the surface of the array at locations where a DNA primer has hybridized to a nucleic acid analyte to produce a duplex nucleic acid. The presence of labeled target nucleic acids on the array surface is then detected and used to determine the presence of the one or more nucleic acid analytes in the sample. Also provided are kits for practicing the subject methods. The subject invention finds use in a variety of different applications, including differential gene expression analysis.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise.

Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

As summarized above, the subject invention provides methods of assaying or testing a sample for the presence of one or more nucleic acid analytes, e.g., mRNA analytes. In further describing the subject invention, the subject methods are discussed first in greater detail, followed by a review of the representative applications in which the subject methods find use and a review of representative kits provided by the invention that can be employed in practicing the subject methods.

### METHODS

As indicated above, the subject invention provides methods of determining the presence of one or more nucleic acid analytes in a sample. In other words, the subject invention provides methods of assaying or testing a sample for the presence of one or more nucleic acid analytes. As such, the subject invention provides methods of detecting the presence of one or more nucleic acid members of a nucleic acid analyte set, where a nucleic acid analyte set is a collection of one or more distinct nucleic acid analytes, e.g., mRNA nucleic acid analytes.

The presence of the one or more nucleic acid analytes in the assayed or tested sample may be determined qualitatively or quantitatively, where the later term includes semi-quantitatively as well as absolute-quantitatively.

In many embodiments, the subject methods are used to assay a sample for at least 10 different nucleic acid analytes, often at least about 100 or more different nucleic acid analytes, including 500, 1000, 10000 or more different nucleic acid analytes.

In practicing the subject methods, a quantity of a fluid sample is contacted with an array of DNA primer compositions, e.g., by depositing a volume of the sample being assayed onto the surface of an array of DNA primer compositions. As will be explained more fully below, contact may be achieved by covering the entire array surface with the sample or by depositing a volume of the sample onto one or more discrete locations of the array surface.

The arrays employed in the subject methods include one or more DNA primers Immobilized onto discrete locations of a surface of a substrate. In other words, the arrays employed in the subject methods include a substrate surface having at least one location thereon occupied by a DNA primer, or composition of primers, that hybridizes to a target of interest, this composition is present on the substrate surface in the form a spot or some other shape. In certain embodiments, the primer composition is homogenous in nature, while in other embodiments, the primer composition is a heterogenous composition.

In many embodiments, the arrays employed in the subject methods have a plurality of distinct DNA primers stably associated with, i.e., immobilized on, a substrate surface, where the plurality of DNA primers is generally known and positioned across the surface of the array in a pattern. Each distinct DNA primer present on the array includes multiple copies of a DNA primer which is distinct from any other DNA primer of any other DNA primer that binds to a different analyte and is present on the substrate surface, where two DNA primers are considered different if they have a different DNA sequence. Typically, any two "different" or "distinct" DNA primers hybridize to different or distinct mRNA molecules, where any two mRNA molecules are considered distinct or different if their sequences have a sequence identity that is less than about 95%, 90%, 85%, 80% or often 75%, as measured using the tool for comparing two sequences using BLAST provided by the National Center for Biotechnology at their Website.

In many embodiments, as mentioned above, each distinct DNA primer is present on the substrate surface in the form of a spot. Typically, the arrays comprise a plurality of spots, where each spot contains a different and distinct DNA primer, i.e. the arrays comprise a plurality of homogenous DNA primer spots. The number of spots on a substrate surface in any given array varies greatly, where the number of spots is at least about 1, usually at least about 10 and more usually at least about 100, and may be as great as 100,000 or greater, but usually does not exceed about 10⁷ and more usually does not exceed about 10⁶. The spots may range in size from about 0.1 µm to 10 mm, usually from about 1 to 1000 µm and more usually from about 10 to 200 µm. The density of the spots may also vary, where the density is generally at least about 1 spot/cm², usually at least about 100 spots/cm² and more usually at least about 400 spots/cm², where the density may be as high as 10⁷ spots/cm² or higher, but generally does not exceed about 10⁶ spots/cm² and usually does not exceed about 10⁵ spots/cm².

A variety of array structures/formats (e.g., substrate format, dimensions, materials, nature of probe attachment, probe pattern layout, etc.) are known to those of skill in the art, where representative array structures include those disclosed or referenced in: U.S. Patent Nos. 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,624,711; 5,639,603; 5,658,734, as well as in WO 93/17126; WO 95/11995; WO 95/35505; EP 742 287; and EP 799 897. Arrays employed in the subject invention may include one or more of the features found in these prior art arrays, so long as they include the DNA primers.

As indicated above, each DNA primer spot present on the substrate surface of the employed arrays includes at least a DNA primer molecule, and more specifically multiple copies of a DNA primer molecule. A feature of the DNA primer molecule that makes up the spot/feature is that it hybridizes to an mRNA molecule of interest under stringent hybridization conditions, where hybridization may in the broadest sense be either to a conserved mRNA sequence, e.g., the polyA tail, or to a variable domain/sequence specific for a give mRNA. In many embodiments, the DNA primer molecule is specific for a single mRNA molecule, where in these embodiments the DNA primer hybridizes to a non-conserved mRNA sequence, i.e., a variable mRNA sequence that is found only in the mRNA of interest. While the length of the DNA primer molecules may vary, in many embodiments, the DNA primer molecules are at least about 10 nt in length, usually at least about 15 nt in length and more usually at least about 25 nt in length, where the DNA primer molecules may be as long as 60 nt or longer. In many embodiments, the length of the DNA primer molecules ranges from about 10 to about 60, usually from about 15 to about 40 and more usually from about 20 to about 35. The amount of DNA primer molecules present in each DNA primer composition is sufficient to provide for a sensitive detection of nucleic acid analyte, and typically ranges from about 1000 molecules/feature to about 100,000 molecules/feature, usually from about 5000 molecules/ feature to about 60,000 molecules/feature. Each DNA primer molecule is immobilized on the substrate surface of the array, where immobilization is achieved by either covalent or non-covalent bonding of the DNA primer molecule to the substrate surface, where any convenient immobilized technology is employed, so long as it provides for immobilization of the DNA primer molecules during use of the array in the subject methods. Of particular interest in certain embodiments are DNA primer arrays in which the DNA primers are covalently attached to the substrate surface, as employed and described in the Experimental Section below.

The arrays of DNA primers can be produced using any convenient protocol, where a number of different protocols are known to those of skill in the art. Of particular interest in certain embodiments are pulse jet fluid deposition protocols coupled in which preformed DNA primers are deposited onto discrete locations of a substrate surface and then covalently bound to the substrate surface, where such protocols are described in U.S. Patent No. 6,221,653 and 6,458,583, and published U.S. Patents Application US 2003027219 and further elaborated below.

In certain embodiments, the arrays employed in the subject methods are those in which two or more distinct regions are present on the surface of the array, where fluid communication between regions is prevented by a barrier means. A variety of barrier means may be present, including raised structures or walls arising from the surface of the array, hydrophobic strips of material positioned on the array surface in a manner sufficient to produce two or more distinct regions, and the like. Such arrays are described in U.S. Patent Nos. 5, 545,531 and 5,807,522.

In certain embodiments of the subject invention, the DNA primer arrays that are employed are arrays in which each DNA primer feature on the array includes, in addition to the DNA primer, one or more additional nucleic acid, e.g., DNA or RNA, synthesis reagents required to enzymatically produce labeled target nucleic acid, i.e., one or more template dependent primer extension reactants. In these embodiments, it is convenient to view each DNA primer feature or spot of the array as a DNA primer composition, where the DNA primer composition includes DNA primer and one or more reagents required for template dependent nucleic acid synthesis, e.g., additional template dependent primer extension reagents required to produce labeled target nucleic acid from a duplex structure made up of a DNA primer hybridized to an analyte nucleic acid, e.g., an mRNA molecule, which serves as template.

In these embodiments, the DNA primer compositions further include at least one of the following additional DNA synthesis reagents:
(1) deoxyribonucleotides, ribonucleotides (for RNA polymerase), and modified nucleotides (e.g., 2,6 diamino purine, etc.) which are incorporated enzymatically into the generated target nucleic acids during practice of the subject methods. Typically, the DNA primer composition includes all four dNTPs, i.e., dATP, dCTP, dGTP and dTTP (and/or dUTP). The dNTPs may be present in varying or equimolar amounts, where the amount of each dNTP typically ranges from about 1 µM to about 2 mM, usually from about 25 µM to about 1 mM and more usually from about 100 µM to about 500 µM ;
(2) at least one type of labeled dNTP, where all four dNTPs may be present in labeled versions or only certain of the dNTPs may be present as labeled dNTPs. The labeled dNTP(s) present in the composition should be labeled with a labeling agent that does not adversely affect to an unacceptable level the incorporation of the labeled dNTP into the enzymatically produced labeled target nucleic acid. Labeled dNTPs of interest include, but are not limited to: dNTPs labeled with isotopic or radioactive labels, such as ³²S, ³² P, ³H, or the like; dNTPs labeled with a fluorescent label, e.g., a cyanine dye, such as Cy3, Cy5, Alexa dyes, such as Alexa 555, 647,Bodipy 630/650; modified dNTPs that contain a reactive group, such as allylamine or biotin, in which a label can be added during a second step and the like. Other labels may also be employed as are known in the art, including labels that are members of a multiple agent signal producing system, and the like. In many embodiments the labeled dNTP is fluorescently labeled. The amount of labeled dNTP(s) may vary so long as it is sufficient to produce detectably labeled target nucleic acids, and in many embodiments ranges from about 1 uM to about 500 uM
(3) a template dependent nucleic acid polymerase, e.g., DNA polymerase, RNA polymerase, etc. A variety of enzymes, usually DNA polymerases, preferably possessing reverse transcriptase activity can be present in the subject DNA primer compositions. Examples of suitable DNA polymerases include the DNA polymerases derived from organisms selected from the group consisting of a thermophilic bacteria and archaebacteria, retroviruses, yeasts, Neurosporas, Drosophilas, primates and rodents. Preferably, the DNA polymerase will be selected from the group consisting of Moloney murine leukemia virus (MMLV) as described in United States Patent No. 4,943,531 and MMLV reverse transcriptase lacking RNaseH activity as described in United States Patent No. 5,405,776 (the disclosures of which patents are herein incorporated by reference), human T-cell leukemia virus type I (HTLV-I), bovine leukemia virus (BLV), Rous sarcoma virus (RSV), human immunodeficiency virus (HIV) and Thermus aquaticus (Taq) or Thermus thermophilus (Tth) as described in United States Patent No. 5,322,770, avian reverse transcriptase, and the like. Suitable DNA polymerases possessing reverse transcriptase activity may be isolated from an organism, obtained commercially or obtained from cells which express high levels of cloned genes encoding the polymerases by methods known to those of skill in the art, where the particular manner of obtaining the polymerase will be chosen based primarily on factors such as convenience, cost, availability and the like. Of particular interest because of their commercial availability and well characterized properties are avian reverse transcriptase and MMLV-RT. Of particular interest in many embodiments is the RNAse H⁻ reverse transcriptase sold under the name SUPERSCRIPT II (Life Technologies, Inc.) The amount of polymerase may vary, but is generally from about 1 U to about 2000 U, usually from about 10 U to about 500 U;
(4) monovalent cations (e.g. Na⁺) and divalent cations (e.g. Mg⁺⁺);
(5) buffers (e.g. Tris), surfactants (e.g. Triton X-100);
(6) RNAase inhibitor and sulfhydril reagents, *e.g.* dithiothreitol; and the like.

The amounts of such additional reagents may vary, where representative amounts are provided in the experimental section, below, so long as the amounts of the above DNA synthesis reagents are effective to provide for the desired labeled target nucleic acid synthesis.

In many of the above embodiments, the DNA primer compositions of each feature include at least two of, and preferably all of the above DNA synthesis reagents mentioned above.

In certain embodiments of the subject invention, the methods include a step of producing an array of DNA primer compositions as described above. The subject arrays of DNA primer compositions may be produced using any convenient protocol. For example, the arrays of DNA primers may be produced by depositing pre-formed primers onto a substrate surface or by chemically synthesizing the primers on a substrate surface, as is known in the art, as described above.

The various reagents can be positioned on the array surface in any convenient order, including before or after contact with the sample, where sample contact is described in greater detail below. For example, each of the reagents can be applied to the surface sequentially, or two or more reagents can be applied at the same time, such that groups of reagents are applied sequentially. Alternatively, a reagent composition that includes all of or at least substantial portion of the reagents can be applied to the surface.

Any convenient manner of positioning the various reagents used in labeled target nucleic acid synthesis may be employed to produce complete DNA primer compositions. Thus, the whole surface may be contacted with reagent compositions. Alternatively, the reagent compositions may be deposited on the surface at the discrete DNA primer locations to produce the DNA primer compositions.

In many embodiments, of interest is the use of pulse jet technology, i.e., pulse-jet fluid deposition, to produce the DNA array and/or deposit the various reagent members of the DNA primer compositions of the array. Pulse jet DNA array fabrication and/or reagent composition deposition protocols are described in U.S. Patent Nos. 4,877,745; 5,073,495; 5,200,051; 5,338,688; 5,474,796; 5,449,754; 5,658,802; 5,700,637; 5,751,839; 5,891,394; 5,958,342, and 6,112,605; 6,221,653; 6,242,266; 6,232,072; and 6,180,351 as well as in published U.S. Patent Application US 2003027219 and in U.S. Patent 6,458,583. Pulse jet technology for deposition of biological agents is also further described in greater detail below.

A feature of using pulse jet deposition protocols for preparation of the DNA primer compositions of the subject arrays is that the deposition process does not result in a substantial modulation of the activity or functionality of the reagent(s) of interest in the fluid that is deposited, despite the small volumes front loaded into the head and the thermal inkjet deposition protocol employed, where the protocol is described in greater detail below. In other words, the overall activity/functionality of the reagents of interest in the fluid that is deposited from the pulse jet during the subject methods is not substantially different from the overall protein activity in the fluid loaded into the pulse prior to deposition. As such, the reagent activity of a quantity of fluid deposited from the pulse jet is substantially the same as that of an identical amount of fluid still present in the pulse jet. In other words, pulse jet deposition of the reagent fluid(s) onto the array surface does not adversely affect the desired protein activity/functionality of the reagent of interest in the fluid.

With respect to protein reagents, e.g., enzymes such as reverse transcriptases, where the sum of all of the individual activities of the individual protein molecules in the deposited volume of fluid is viewed as the overall protein activity of the fluid for the deposited volume of fluid, the deposition process does not substantially change the overall protein activity of the deposited fluid sample, if at all, because the deposition process does not modify a significant percentage of the total number of protein molecules present in the deposited fluid sample. Since a significant percentage of the total number of protein molecules in the quantity of deposited fluid is not modified by deposition according to the subject methods, the total percentage of protein molecules that are modified, e.g., denatured, degraded or otherwise inactivated etc., at least partially or completely, by the deposition process does not exceed about 10%, usually does not exceed about 5% and more usually does not exceed about 1 %. In terms of concentration of the active protein of interest, any change in concentration of the activity or function protein of interest in the sample that occurs in the deposited fluid does not exceed about 20%, usually does not exceed about 10% and more usually does not exceed about 5%.

In terms of the overall protein activity, the amount of modulation, if any, that occurs because of the manner of deposition is typically less than about 10 %, usually less than about 5 % and more usually less than about 1 %. A convenient means of determining the amount of change in overall protein activity caused by deposition is to compare the protein activity of a quantity of fluid that has been expelled or fired from the inkjet to the protein activity of the same quantity of an identical fluid that has not been expelled or fired, e.g., loaded fluid still in the head. The particular assay that is employed to achieve the above comparison necessarily varies depending on the particular nature of the protein and activity/functionality of interest.

In certain embodiments, the protein activity or functionality that is preserved in the deposited quantity of fluid is an enzymatic activity. In these embodiments, any change in activity, e.g., decrease, in enzymatic activity that is observed in the deposited fluid as compared to the predeposited fluid is not substantial, such that it does not exceed about 10%, usually does not exceed about 5% and more usually does not exceed about 1 %.

In certain embodiments where the DNA primer compositions are produced using one or more pulse jet fluid deposition steps, it may be desirable to prevent evaporation of the fluid sample following deposition. Evaporation may be prevented in a number of different ways. The subject methods may be carried out in a high humidity environment. By "high humidity" is meant an environment in which the humidity is at least about 86 % relative humidity, usually at least about 95 % relative humidity and more usually at least about 99% relative humidity. Alternatively, one may apply an evaporation retarding agent, e.g. mineral oil, glycerol solution, polyethylene glycol, etc., over the surface of the deposited sample, e.g. by using a thermal inkjet as described above.

In yet other embodiments, it may be desired to rapidly dehydrate the deposited sample following deposition, e.g., where it is desired to produce a dry deposited sample on the substrate surface, e.g., for storage prior to use. By depositing the fluid sample in a dry environment and a suitable temperature, the water component of the deposited fluid sample rapidly evaporates, leaving active protein that can be readily stored for subsequent use. In these embodiments, the relative humidity of the environment typically does not exceed about 35%, usually does not exceed about 20% and more usually does not exceed about 10%. The temperature typically ranges from about 2°C to about 30°C, usually from about 4 °C to about 25°C and more usually from about 10°C to about 20°C.

Where desired, following deposition of the desired amount of reagent fluid, the head may be washed and front loaded with another reagent containing fluid for subsequent fluid deposition. Washing of the head can be accomplished using any convenient protocol, e.g., via front loading and expelling an appropriate wash buffer, one or more times, by backloading and expelling an appropriate wash buffer, etc. In addition, the head may be manually or automatically wiped clean to remove any sample/wash solution left from the previous deposition.

In many embodiments, the head is rapidly washed and reloaded with a new solution, such that the time period starting from the deposition of the first fluid to the loading of the second fluid, i.e., the washing time, is extremely short. In these embodiments, the wash time typically does not exceed about 1 minute, usually does not exceed about 5 minutes and more usually does not exceed about 30minutes. The wash protocol in these embodiments may include a single flushing or multiple flushes, where the total number of flushes will typically not exceed about 3, usually will not exceed about 5 and more usually will not exceed about 10. The wash fluid employed in these embodiments is typically one that provides for removal of substantially all proteins of the first fluid in a minimal number of flushes, where representative fluids of interest include, but are not limited to: saline buffer solution with surfactant, and the like.

The above methods can be substantially, if not completely automated, so that fluid can be loaded and deposited onto a surface automatically. As such, the subject methods are amenable to high throughput applications, e.g., high throughput manufacturing applications. Such automatic devices comprise at least a means for precisely controlling the position of the head with respect to an array surface (an XYZ translational mechanism) and for firing the head. Such automated devices are described, for example, in U.S. Patents 6,242,266; 6,232,072; and 6,180,351. In certain embodiments, a pre-prepared array of DNA primer compositions is employed to assay a given sample. In many of these embodiments, the DNA primer composition array is an array that is present in a dry, storage stable format. By dry, storage stable format is meant an array that is present in dry form, where the various reagents compositions making up the array are dry, i.e., are not fluid compositions. In many of these embodiments, the subject DNA primer compositions include one or more reagent activity preservation agents, where such agents include, but are not limited to: trehalose, and the like. A representative example of a DNA primer array present as a dry composition, as well as its preparation, is provided in the experimental section, below.

In practicing the subject methods, the DNA primer array, and more specifically the DNA primer features/spots of the array, (which may or may not be a DNA primer composition array depending on whether the DNA synthesis reagents are or are not present prior to sample contact) is contacted with the sample to be assayed. The fluid sample that is contacted with the array surface according to the subject invention is a fluid sample that is suspected of containing one or more nucleic acid analytes of interest. In other words, the fluid sample may or may not actually contain the analyte(s) of interest, where the purpose of the array-based assays in which the methods of the subject invention find use is to determine whether or not the sample has the analyte(s) of interest. The analyte(s) of interest is a nucleic acid, e.g., an oligonucleotide, polynucleotide, etc, and in many embodiments is mRNA.

The fluid sample is generally derived from a physiological fluid, e.g. naturally occurring fluids, such as plasma, tears, urine, etc., derivatives of cells or tissues, e.g. cell lysates, etc., where the fluid may or may not be pre-treated to produce the sample. Examples of pre-treatments to which the original fluid may be subjected in order to produce the sample include dilution, concentration, labeling, denaturation, etc., where such protocols are well known to those of skill in the art. Generally, the fluid sample is an aqueous fluid sample, where the fluid sample may or may not include one or more additional agents, such as co-solvents, buffering salts, surfactants, ions, denaturants, enzymes etc.

As indicated above, the fluid sample contacted with the array surface in the subject methods is a nucleic acid fluid sample. By nucleic acid fluid sample is meant a fluid sample that contains a nucleic acid, usually a plurality of distinct nucleic acids, where the nucleic acids may be oligonucleotides, polynucleotides, e.g., mRNAs or derivatives thereof, e.g., cDNAs, amplified RNAs,etc. In many preferred embodiments, the nucleic acid fluid sample contains a plurality of distinct mRNAs from a physiological sample, e.g. cell or tissue of interest.

The fluid sample is contacted with the array surface using any convenient protocol. As such, protocols may be employed where the entire array is contacted with the fluid sample being assayed. For example, the array surface may be flooded with the sample being assayed, the array surface may be immersed in the sample being assayed etc. Alternatively, fluid sample may be deposited onto discrete locations of the array surface, where in these embodiments a volume of the fluid sample is deposited onto the various DNA primer compositions of the array surface.

Where one is assaying a sample of limited volume, methods of depositing extremely small quantities of the fluid sample, e.g. a pico liter volume of the fluid sample, onto the surface of the array, are of interest. By "pico liter quantity" is meant a volume of fluid that is at least about .05 pl, usually at least about 0.1 pl and more usually at least about 1 pl, where the volume may be as high as 250 pl or higher, but generally does not exceed about 10 µL and usually does not exceed about 1 µl.

One type of fluid deposition protocol of interest that may be employed in these embodiments is the pulse-jet deposition protocol discussed above, where a pulse jet device, such as a thermal or piezoelectric pulse jet fluid deposition device, is employed. In many embodiments, the pulse jet device that is employed is a thermal pulse jet device.

As is known to those of skill in the art, thermal pulse jet fluid deposition devices typically have at least the following components: (a) an orifice; (b) a firing chamber; and (c) a heating element. Thermal pulse-jet fluid deposition devices and methods for their manufacture and use are described in a number of different U.S. Patents, including: 5,772,829; 5,745,128; 5,736,998; 5,736,995; 5,726,690; 5,714,989; 5,682,188; 5,677,577; 5,642,142; 5,636,441; 5,635,968; 5,635,966; 5,595,785; 5,477,255; 5,434,606; 5,426,458; 5,350,616; 5,341,160; 5,300,958; 5,229,785; 5,187,500; 5,167,776; 5,159,353; 5,122,812; and 4,791,435.

Thermal pulse jet fluid deposition devices finding use in the subject methods will generally have the following characteristics. The size of the orifice is sufficient to produce a spot of suitable dimensions on the substrate surface (described in greater detail above), where the orifice generally has a diameter (or exit diagonal depending on the specific format of the ink jet head) ranging from about 1 to 1000 µm, usually from about 5 to 100 µm and more usually from about 10 to 60 µm. The firing chamber has a volume ranging from about 1 pl to 10 nl, usually from about 10 pl to 5 nl and more usually from about 50 pl to 1.5 nl. The heating element will preferably be made out of a material that can deliver a quick energy pulse, where suitable materials include: TaAl and the like. The thermal element is capable of achieving temperatures sufficient to vaporize a sufficient volume of the nucleic acid composition in the firing chamber to produce a bubble of suitable dimensions upon actuation of the head. Generally, the heating element is capable of attaining temperatures of at least about 100 °C, usually at least about 400°C and more usually at least about 700 °C, where the temperature achievable by the heating element may be as high as 1000°C or higher. The device may also have one or more reservoirs. In other words, the device may be a single reservoir device or a multi-reservoir device. When present, the reservoir will typically have a volume ranging from about 1 pl to 1 ml, usually from about .5 µl to 10 µl and more usually from about 1 µl to 5 µl. A variety of thermal inkjet heads are available commercially, where such devices include: the HP92261A thermal inkjet head (available from Hewlett-Packard Co., Palo Alto CA), the HP 51645A thermal inkjet head (available from Hewlett-Packard Co. Palo Alto CA), the inkjet produced by (Cannon Kabushiki Kaisha,Tokyo, Japan) and the like. Specific inkjet heads of interest are disclosed in U.S. Patent Nos. 5,736,998 and 4,668,052.

In practicing the subject methods, the thermal pulse jet device is loaded with a fluid sample, e.g., a nucleic acid fluid sample. The fluid may be loaded into the firing chamber and fluid reservoir using any convenient means. Thus, conventional methods of introducing ink into thermal inkjet heads may be employed. Where such methods are employed, following loading of the fluid sample into the inkjet head, it is often desirable to "prime" the device prior to use. One means of priming the device is to apply sufficient pressure to the fluid in the reservoir (or conversely negative pressure to the orifice) such that a volume of fluid is forced out of the orifice. Such priming methods are currently employed in the printing industry and thus are well known to those of skill in the art.

Alternatively, where minimal waste of the fluid sample desired, e.g., where the fluid is an expensive or rare mRNA sample, the following method of loading the fluid sample into the firing chamber and reservoir may be employed. In this method of fluid sample loading, the orifice is contacted with the fluid under conditions sufficient for fluid to flow through the orifice and into the firing chamber of the head, where fluid flow is due, at least in part, to capillary forces. To assist in the flow of fluid into the orifice, back pressure in the form of suction (i.e. negative pressure) may be applied to the firing chamber (and reservoir, if present) of the head, where the back pressure will typically be at least about 5, and may be at least about 10 and even as great as about 100 inches of H₂O or more.

To deposit fluid onto the surface of an array according to the subject methods, the fluid sample loaded thermal pulse jet head is positioned in opposing relationship relative to the surface of the array (e.g. with an XYZ translational means), where the orifice is in opposition to the position on the array surface at which deposition of the nucleic acid is desired (e.g. opposite a binding agent spot on the array). The distance between the orifice and the array surface will not be so great that the volume of nucleic acid fluid cannot reach the array surface and produce a spot in a reproducible manner. As such, the distance between the orifice and the array surface will generally range from about 10 µm to 10 mm, usually from about 100 µm to 2 mm and more usually from about 200 µm to 1 mm.

After the head is placed into position relative to the array surface, the temperature of the heating element or resistor of the head is raised to a temperature sufficient to vaporize a portion of the fluid immediately adjacent to the resistor and produce a bubble. In raising the temperature of the heating element, the temperature of the heating element is raised to at least about 100 °C, usually at least about 400 °C and more usually at least about 700 °C, where the temperature may be raised as high as 1000 °C or higher, but will usually be raised to a temperature that does not exceed about 2000 °C and more usually does not exceed about 1500 °C. As such, a sufficient amount of energy will be delivered to the resistor to produce the requisite temperature rise, where the amount of energy will generally range from about 1.0 to 100 µJ, usually from about 1.5 to 15 µJ. The portion of fluid in the firing chamber that is vaporized will be sufficient to produce a bubble in the firing chamber of sufficient volume to force an amount of liquid out of the orifice.

The formation of the bubble in the firing chamber traps a portion or volume of the fluid present in the firing chamber between the heating element and the orifice and forces an amount or volume of fluid out of the orifice at high speed. The amount or volume of fluid that is forced out of the firing chamber can be controlled depending on the specific amount of fluid that is desired to be deposited on the substrate. As is known in the art, the amount of fluid that is expelled can be controlled by changing one or more of a number of different parameters of the ink jet head, including: the orifice diameter, the orifice length (depth), the size of the firing chamber, the size of the heating element, and the like. Such variations are well known to those of skill in the art. As such, the amount or volume of fluid that is forced out or expelled from the firing chamber may range from about 0.1 to 2000 pl, usually from about 0.5 to 500 pl and more usually from about 1.0 to 250 pl. The speed at which the fluid is expelled from the firing chamber is at least about 1 m/s, usually at least about 10 m/s and may be as great as about 20 m/s or greater.

Upon actuation of the thermal pulse jet head, as described above, fluid is expelled from the orifice and travels to the array surface. Upon contact with the array surface, the fluid combines with the DNA primer composition located at the position of the array on which the fluid is deposited. The deposited fluid typically forms a spot on the array surface. As mentioned above, by varying the design parameters of the thermal pulse jet head, the spot dimensions can be controlled such that spots of various sizes can be produced. With the subjects methods, one can produce spot sizes which have diameters ranging from a minimum of about 0.1 µm to a maximum of about 10 mm. In those embodiments where very small spot sizes are desired, one can produce small spots that have a diameter ranging from about 0.1 µm to 1.0 mm, usually from about 1.0 µm to 500 µm and more usually from about 10 µm to 200 µm. In many embodiments, the spot sizes range from about 30 to 100 µm.

An important feature of the subject invention is that the deposition process does not adversely affect the binding capabilities of the nucleic acid analyte(s) (if present) in the sample with its respective binding pair member present on the array. For example, the deposited nucleic acid is capable of hybridizing to complementary DNA primer molecules present on the array. In other words, the deposition process does not adversely affect the nucleic acid of the sample, e.g. does not physically alter the nature of the nucleic acid such that it cannot subsequently participate in Watson-Crick type hydrogen bonding interactions.

In certain embodiments, it may be desirable to prevent evaporation of the fluid sample following deposition. Evaporation may be prevented in a number of different ways. The subject methods may be carried out in a high humidity environment. By "high humidity" is meant an environment in which the humidity is at least about 86 % relative humidity, usually at least about 95 % relative humidity and more usually at least about 99% relative humidity. Alternatively, one may apply an evaporation retarding agent, e.g. mineral oil, glycerol solution, polyethylene glycol, etc., over the surface of the deposited sample, e.g. by using a thermal inkjet as described above.

Where desired, following deposition of the desired amount of fluid sample, the head may be washed and front loaded with another fluid sample for subsequent fluid deposition, e.g., on a different array. Washing of the head can be accomplished using any convenient protocol, e.g., via front loading and expelling an appropriate wash buffer, one or more times, by backloading and expelling an appropriate wash buffer, etc. In addition, the head may be manually or automatically wiped clean to remove any sample/wash solution left from the previous deposition.

The above methods can be substantially, if not completely automated, so that fluid can be loaded and deposited onto a surface automatically. As such, the subject methods are amenable to high throughput applications, e.g., high throughput manufacturing applications. Such automatic devices comprise at least a means for precisely controlling the position of the head with respect to an array surface (an XYZ translational mechanism) and for firing the head. Such automated devices are disclosed in, for example, in U.S. Patents 6,242,266; 6,232,072; and 6,180,351.

Regardless of how the fluid sample is deposited onto the substrate surface, the sample is contacted with the array under conditions that produce duplex nucleic acid structures at those locations on the array where a DNA primer is present that is the complement of a nucleic acid analyte in the sample, e.g., an mRNA of the sample. Typically, the array surface is contacted with the sample under stringent hybridization conditions that are compatible with the DNA polymerase to produce duplexes on the array surface between perfectly matched DNA primers and their corresponding nucleic acid analytes that are present in the sample, e.g., their corresponding mRNA analytes present in the sample. The hybridization conditions typically may be the optimized reaction conditions for the DNA polymerase. An example of stringent hybridization conditions is 42°C and 50 mM Tris-HCl pH 8.3 and 75 mM KCI. Other stringent hybridization conditions are known in the art and may also be employed to identify nucleic acids of this particular embodiment of the invention.

Following contact and production of duplex structures at locations of the array where nucleic acid analytes present in the sample hybridize to complementary DNA primers of DNA primer compositions, the resultant array is maintained under conditions sufficient to produce labeled target nucleic acid where the duplex structures are present. Any convenient conditions may be employed, e.g., where representative conditions include first strand cDNA synthesis conditions, which conditions are known to those of skill in the art. Typically, the array is maintained at a temperature that ranges from about 4 to about 60, usually from about 20 to about 45 for a period of time ranging from about 30 min to about 10 hours, usually from about 1 hour to about 4 hours. Since the array is maintained under conditions sufficient to produce labeled target nucleic acid at those locations of the array where a primer/analyte duplex structure is present, each DNA primer spot or feature of the array includes, in addition to DNA primer and sample, DNA synthesis reagents, where the DNA synthesis reagents include:
(1) deoxyribonucleotides which are incorporated into the enzymatically generated target nucleic acids during practice of the subject methods. Typically, the DNA primer composition includes all four dNTPs, i.e., dATP, dCTP, dGTP and dTTP. The dNTPs may be present in varying or equimolar amounts, where the amount of each dNTP typically ranges from about 1 uM to about 2 mM, usually from about 25 uM to about 1 mM and more usually from about 100 uM to about 500 uM;
(2) at least one type of labeled dNTP, where all four dNTPs may be present in labeled versions or only certain of the dNTPs may be present as labeled dNTPs. The labeled dNTP(s) present in the composition should be labeled with a labeling agent that does not adversely affect to an unacceptable level the incorporation of the labeled dNTP into the enzymatically produced labeled target nucleic acid. Labeled dNTPs of interest include, but are not limited to: dNTPs labeled with isotopic or radioactive labels, such as ³²S, ³² P, ³H, or the like; dNTPs labeled with a fluorescent label, e.g., a cyanine dye, such as Cy3, Cy5, Alexa dyes, such as Alexa 555 and Alexa 647, Bodipy 630/650; modified dNTPS that contain a reactive group, such as allylamine or biotin, that can be labeled in a second reaction, and the like. Other labels may also be employed as are known in the art, including labels that are members of a multiple agent signal producing system, and the like. In many embodiments the labeled dNTP is fluorescently labeled. The amount of labeled dNTP(s) may vary so long as it is sufficient to produce detectably labeled target nucleic acids, and in many embodiments ranges from about 1 uM to about 200 uM;
(3) a template dependent DNA polymerase. A variety of enzymes, usually DNA polymerases, preferably possessing reverse transcriptase activity can be present in the subject DNA primer compositions. Examples of suitable DNA polymerases include the DNA polymerases derived from organisms selected from the group consisting of a thermophilic bacteria and archaebacteria, retroviruses, yeasts, Neurosporas, Drosophilas, primates and rodents. Preferably, the DNA polymerase will be selected from the group consisting of Moloney murine leukemia virus (MMLV) as described in United States Patent No. 4,943,531 and MMLV reverse transcriptase lacking RNaseH activity as described in United States Patent No. 5,405,776, human T-cell leukemia virus type I (HTLV-I), bovine leukemia virus (BLV), Rous sarcoma virus (RSV), human immunodeficiency virus (HIV) and Thermus aquaticus (Taq) or Thermus thermophilus (Tth) as described in United States Patent No. 5,322,770, avian reverse transcriptase, and the like. Suitable DNA polymerases possessing reverse transcriptase activity may be isolated from an organism, obtained commercially or obtained from cells which express high levels of cloned genes encoding the polymerases by methods known to those of skill in the art, where the particular manner of obtaining the polymerase will be chosen based primarily on factors such as convenience, cost, availability and the like. Of particular interest because of their commercial availability and well characterized properties are avian reverse transcriptase and MMLV-RT. Of particular interest in many embodiments is the RNAse H⁻ reverse transcriptase sold under the name SUPERSCRIPT II (Life Technologies, Inc.) The amount of polymerase may vary, but is generally from about 1 U to about 1000 U, usually from about 10 U to about 400 U;
(4) monovalent cations (e.g. Na⁺) and divalent cations (e.g. Mg⁺⁺);
(5) buffers (e.g. Tris), surfactants (e.g. Triton X-100);
(6) RNAase inhibitor and sulfhydril reagents, *e.g*. dithiothreitol; and the like. The amounts of the above reagents may vary, where representative amounts are provided in the experimental section, below, so long as the amounts of the above DNA synthesis reagents are effective to provide for the desired labeled target nucleic acid synthesis.

The above steps result in the enzymatic production of labeled target nucleic acids on the surface of the array substrate at locations where DNA primers of the DNA primer compositions have hybridized to nucleic acid analytes present in the sample. In other words, the above steps result in the production of labeled target nucleic acids in those locations of the array that correspond to specific analytes of interest that are present in the sample.

Following enzymatic production of labeled target nucleic acids on the substrate surface of the array and washing of the array, the array is then read to detect the presence and location of the enzymatically produced labeled target nucleic acids. Where the labeled target nucleic acids are fluorescently labeled, reading of the array may be accomplished by illuminating the array and reading the location and intensity of resulting fluorescence at each feature of the array to detect any binding complexes on the surface of the array. For example, a scanner may be used for this purpose that is similar to the AGILENT MICROARRAY scanner available from Agilent Technologies, Palo Alto, CA. Other suitable apparatus and methods are described in published U.S. patent application 20020160369 "Reading Multi-Featured Arrays" by Dorsel et al.; and U.S. patent 6,406,849 "Interrogating Multi-Featured Arrays" by Dorsel et al.

However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample).

Once the results, i.e., assay data, are obtained, the results are employed to determine the presence of the nucleic acid analytes in the assay sample. In other words, the presence of the analyte(s) in the sample is then deduced from the detection of labeled target nucleic acids on the substrate surface, where the location of a given labeled target nucleic acid imparts information about the identity of the corresponding nucleic acid analyte and the intensity of the signal may impart information regarding the quantity of the corresponding nucleic acid analyte in the sample.

In certain embodiments, the subject methods include a step of transmitting data from at least one of the detecting and deriving steps, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

### UTILITY

The subject methods find use in a variety of different array-based applications. Array-based applications in which the subject invention finds use include: differential expression analysis, gene discovery, and the like. A variety of array-based applications are described in: U.S. Patent Nos. 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,624,711; 5,639,603; 5,658,734; as well as WO 93/17126; WO 95/11995; WO 95/35505; EP 742 287; and EP 799 897.

### KITS

Finally, kits for use in practicing the subject methods are provided. The subject kits at least include one or more DNA primer compositions arrays, or precursors thereof, e.g., DNA primer arrays, as described above. The kits may further include one or more additional components necessary for carrying out the subject methods, such as sample preparation reagents, buffers, labels, and the like. As such, the kits may include one or more containers such as vials or bottles, with each container containing a separate component for the assay, such as an array, and reagents for carrying out nucleic acid hybridization assays according to the invention, reagents for producing DNA primer compositions, etc. Thus, the kit will comprise in packaged combination, an array, wherein the array comprises DNA primer compositions or precursors thereof. The kits may also include a denaturation reagent for denaturing the analyte, hybridization buffers, wash solutions, enzyme substrates, negative and positive controls and written instructions for carrying out the assay.

Finally, the kits may further include instructions for using the subject devices in the subject methods . The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

The following examples are offered by way of illustration and not by way of limitation.

### EXPERIMENTAL

### Example I.

A glass slide containing inkjet deposited cDNAs that have been partially denatured and crosslinked to the surface is used. Cy5-dCTP is hand spotted randomly onto the surface and allowed to dry. A solution containing buffer, sequence-specific primers, and dNTPs and a second solution containing DNA polymerase is loaded into an inkjet and fired onto the glass slide. The slide is incubated in a humid chamber at 37 °C for 60 minutes to allow DNA polymerization. The slide is washed to remove unincorporated Cy5-dCTP. The slide is then scanned for covalently linked Cy5-dCMP to the DNA attached to the surface, indicating that the DNA polymerase synthesized DNA. The results show that multiple reagents may be deposited onto the surface using the subject methods. The results also show that the activity of the polymerase enzyme is maintained during the deposition process.

### Example II.

A. Oligonucleotide DNA primers are loaded into the ink jet head and spotted onto a derivatized glass support capable of binding and immobilizing DNA via an amine at the 5'-end of the primer. The primers are spotted onto the surface at defined locations on the glass substrate in order to generate an array of primers across the surface, with the 3'-OH of the primer accessible and the 5'-end of the primer covalently attached to the surface. The glass substrate contains fiducials that allow for repeated positioning of the ink jet head at defined locations. The glass substrate containing the array of DNA primers is removed and processed for covalent attachment of the primers and passivation of the non-coupled oligo reactive sites. The glass substrate is placed again on the XY stage under the inkjet device. The fiducials on the glass are used to orient the glass in the same position as when the primers were spotted. A 1 mM solution of dNTPs is transferred to a reservoir, a reaction buffer of 250 mM Tris-HCI, pH 8.3, 15 mM MgCl₂, 50 mM DTT to a second reservoir, a 1 mM solution of Cy3-dCTP is delivered to a third reservoir, and an RNA sample (100 ng/uL) is transferred to a fourth reservoir. A DNA polymerase, such as MMLV RT at 200 units/uL, diluted into an excipient like trehalose at a concentration of 0.6M, to give a final concentration of 20 units/uL is transferred to another reservoir of the ink jet head. As the ink jet travels across the array, individual nozzles are fired from the ink jet head, delivering dNTPs, buffer, Cy3-dCTP, RNA, and polymerase to each location of previously spotted DNA primers. Following deposition of the reagents, the array of reactions is transferred to a humidified incubator (42°C) for 60 min. while DNA synthesis occurs. The glass substrate is then washed in a low ionic strength buffer at neutral pH, such as 10 mM Tris-HCl, pH 7.2 containing 0.1% SDS. The substrate is further washed in deionized water, and the amount of fluorescence in each location is determined by scanning the array on a DNA microarray scanner, such as the Agilent microarray scanner. The fluorescence intensity is proportional to the amount of RNA present in the sample that contains the specific sequence corresponding to each primer sequence.
B. Oligonucleotide DNA primers are loaded into the ink jet head and spotted onto a derivatized glass support capable of binding and immobilizing DNA via an amine at the 5'-end of the primer. The primers are spotted onto the surface at defined locations on the glass substrate in order to generate multiple small arrays across the surface. The small arrays are themselves arrayed across the substrate and identical with respect to oligo layout to other arrays on the substrate. The small arrays are spatially separated from each other creating zones where no oligo nucleotides are present. These zones are 5mm wide. The glass substrate contains fiducials that allow for repeated positioning of the ink jet head at defined locations. The glass substrate containing the array of DNA primers is removed and processed for covalent attachment of the primers and passivation of the non-coupled oligo reactive sites. The glass substrate is placed again on the XY stage under the inkjet device. The fiducials on the glass are used to orient the glass in the same position as when the primers were spotted. A 1 mM solution of dNTPs is transferred to a reservoir, a reaction buffer of 250 mM Tris-HCI, pH 8.3, 15 mM MgCl₂, 50 mM DTT to a second reservoir, and a 1 mM solution of Cy3-dCTP is delivered to a third reservoir. A DNA polymerase, such as MMLV RT at 200 units/uL, diluted into an excipient like trehalose at a concentration of 0.6M, to give a final concentration of 20 units/uL is transferred to another reservoir of the ink jet head. As the ink jet travels across the array, individual nozzles are fired from the ink jet head, delivering dNTPs, buffer, and Cy3-dCTP. Each droplet is allowed to dry. Next the DNA polymerase is delivered to each location of previously spotted DNA primers. The dispensed volume of 30 pl is allowed to dry. The presence of the trehalose stabilizes the polymerase. Following deposition of the reagents, the array of reactions is transferred to a dry environment (N₂) containing no more than 12% humidity.

An RNA sample extracted from a tissue of interest is heat denatured and delivered to one of the small arrays in a volume sufficient to cover the array. The substrate is incubated in a humidified incubator (42°C) for 60 min. while DNA synthesis occurs. The glass substrate is then washed in a low ionic strength buffer at neutral pH, such as 10 mM Tris-HCl, pH 7.2 containing 0.1% SDS. The substrate is further washed in deionized water, and the amount of fluorescence in each location is determined by scanning the array on a DNA microarray scanner, such as the Agilent microarray scanner. The fluorescence intensity is proportional to the amount of RNA present in the sample that contains the specific sequence corresponding to each primer sequence.

It is evident from the above results and discussion that a simple and efficient way to perform array based nucleic acid analyte detection assays, such as differential gene expression assays, is provided. Benefits of the subject invention include the ability to employ small volumes of sample. In addition, the fluid deposition embodiments of the subject invention have the benefit of using extremely small quantities of reagents and sample, providing efficiencies in terms of resource use. Additional benefits include the ability to automate the subject methods and readily adapt them to high throughput formats. As such, the subject invention is a significant contribution to the art.

The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

## Claims

1. A method of producing an array for use in assaying a sample for the presence of one or more nucleic acid analyte members of a nucleic acid analyte set, said method comprising, in any convenient order:
(A) providing a solid support;
(B) immobilising at least two distinct DNA primer compositions on a surface of the solid support at distinct locations; wherein each of the distinct DNA primer compositions comprises a DNA primer that hybridises under stringent conditions to a different member of said nucleic acid analyte set; and
(C) depositing a polymerase at the same distinct locations on the surface of the solid support using pulse-jet technology.

2. A method as claimed in claim 1, wherein the DNA primer compositions further include:
(i) dATP;
(iii) dGTP;
(iii) dTTP;
(iv) dUTP;
(v) dCTP; or
(vi) at least one type of labeled dNTP.

3. A method as claimed in claim 1 or 2, wherein said polymerase includes an activity which is: a DNA polymerase, an RNA polymerase or a reverse transcriptase.

4. A method as claimed in any of claims 1 to 3, wherein each of said primer compositions further includes an RNAse inhibitor.

5. A method as claimed in any preceding claim, wherein the array of DNA primer compositions is in a dry, storage stable format, wherein each DNA primer composition includes:
(a) a DNA primer; and
(b) at least one of an effective amount of a DNA synthesis reagent which is:
(i) dATP;
(iii) dGTP;
(iii) dTTP;
(iv) dCTP; or
(v) at least one type of labeled dNTP:

6. An array obtainable by the method of any claims 1 to 5.

7. A method of assaying a sample for the presence of one or more nucleic acid analyte members of a nucleic acid analyte set, said method comprising:
(a) providing an array as claimed in claim 6;
(b) contacting each of said at least two distinct DNA primer compositions of said array with said sample under DNA synthesis conditions sufficient to produce labeled target nucleic acids at locations on said surface where a nucleic acid analyte present in said sample hybridizes to a DNA primer to produce a duplex nucleic acid;
(c) detecting the presence of labeled target nucleic acids on said array surface to obtain assay data; and
(d) employing said assay data to determine the presence of one or more nucleic acid analytes in said sample.

8. A method as claimed in Claim 7, wherein said method is a method of quantitatively determining the presence of said one or more nucleic acid analytes.

9. A method as claimed in Claim 7 or 8, wherein said method comprises sequentially contacting said each of said primer compositions with one or more additional template dependent primer extension reactants and said sample.

10. A method as claimed in any preceding claim, wherein said contacting step comprises depositing a volume of sample on each DNA primer of said array.

11. A method as claimed in Claim 10, wherein said volume of sample is deposited by pulse-jet fluid deposition.

12. A method as claimed in any preceding claim, wherein said contacting step comprises contacting said entire array surface with said sample.

## Patentansprüche

1. Ein Verfahren zum Erzeugen eines Arrays zur Verwendung beim Untersuchen einer Probe auf das Vorliegen eines oder mehrerer Nucleinsäure-Analytglieder eines Nucleinsäure-Analytsatzes, wobei das Verfahren in einer beliebigen zweckmäßigen Reihenfolge folgende Schritte umfasst:
(A) Bereitstellen eines Feststoffträgers;
(B) Immobilisieren zumindest zweier gesonderter DNA-Startermolekül-Zusammensetzungen auf einer Oberfläche des Feststoffträgers an gesonderten Stellen; wobei jede der gesonderten DNA-Startermolekül-Zusammensetzungen ein DNA-Startermolekül umfasst, das unter strengen Bedingungen zu einem anderen Glied des Nucleinsäure-Analytsatzes hybridisiert; und
(C) Aufbringen einer Polymerase an denselben gesonderten Stellen auf der Oberfläche des Feststoffträgers unter Verwendung einer Pulsstrahltechnologie.

2. Ein Verfahren gemäß Anspruch 1, bei dem die DNA-Startermolekül-Zusammensetzungen ferner folgende umfassen:
(i) dATP;
(ii) dGTP;
(iii) dTTP;
(iv) dUTP;
(v) dCTP; oder
(vi) zumindest eine Art eines markierten dNTP.

3. Ein Verfahren gemäß Anspruch 1 oder 2, bei dem die Polymerase eine Aktivität umfasst, die: eine DNA-Polymerase, eine RNA-Polymerase oder eine Revertase ist.

4. Ein Verfahren gemäß einem der Ansprüche 1 bis 3, bei dem jede der Startermolekül-Zusammensetzungen ferner einen Ribonuclease-Hemmstoff umfasst.

5. Ein Verfahren gemäß einem der vorhergehenden Ansprüche, bei dem das Array von DNA-Startermolekül-Zusammensetzungen in einem trockenen, speicherungsstabilen Format vorliegt, wobei jede DNA-Startermolekül-Zusammensetzung Folgendes umfasst:
(a) ein DNA-Startermolekül; und
(b) zumindest eines einer effektiven Menge eines DNA-Synthese-Reagens, das Folgendes ist:
(i) dATP;
(ii) dGTP;
(iii) dTTP;
(iv) dCTP; oder
(v) zumindest eine Art eines markierten dNTP.

6. Ein Array, das anhand des Verfahrens gemäß einem der Ansprüche 1 bis 5 erhältlich ist.

7. Ein Verfahren zum Untersuchen einer Probe auf das Vorliegen eines oder mehrerer Nucleinsäure-Analytglieder eines Nucleinsäure-Analytsatzes, wobei das Verfahren folgende Schritte umfasst:
(a) Bereitstellen eines Arrays gemäß Anspruch 6;
(b) Kontaktieren jeder der zumindest zwei gesonderten DNA-Startermolekül-Zusammensetzungen des Arrays mit der Probe unter DNA-Synthese-Bedingungen, die ausreichend sind, um markierte Zielnucleinsäuren an Stellen auf der Oberfläche zu erzeugen, wo ein in der Probe vorhandener Nucleinsäureanalyt zu einem DNA-Startermolekül hybridisiert, um eine Doppelnucleinsäure zu erzeugen;
(c) Erfassen des Vorliegens markierter Zielnucleinsäuren auf der Arrayoberfläche, um Untersuchungsdaten zu erhalten; und
(d) Verwenden der Untersuchungsdaten, um das Vorliegen eines oder mehrerer Nucleinsäureanalyten in der Probe zu ermitteln.

8. Ein Verfahren gemäß Anspruch 7, wobei das Verfahren ein Verfahren zum quantitativen Ermitteln des Vorliegens des einen oder der mehreren Nucleinsäureanalyte ist.

9. Ein Verfahren gemäß Anspruch 7 oder 8, wobei das Verfahren ein sequentielles Kontaktieren jeder der Startermolekül-Zusammensetzungen mit einem oder mehreren zusätzlichen matrizenabhängigen Startermolekül-Ausdehnungsreaktanten und der Probe umfasst.

10. Ein Verfahren gemäß einem der vorhergehenden Ansprüche, bei dem der Kontaktierungsschritt ein Aufbringen eines Volumens einer Probe auf jedes DNA-Startermolekül des Arrays umfasst.

11. Ein Verfahren gemäß Anspruch 10, bei dem das Volumen der Probe mittels einer Pulsstrahl-Fluidaufbringung aufgebracht wird.

12. Ein Verfahren gemäß einem der vorhergehenden Ansprüche, bei dem der Kontaktierungsschritt ein Kontaktieren der gesamten Arrayoberfläche mit der Probe umfasst.

## Revendications

1. Procédé pour produire un arrangement destiné' à être utilisée pour analyser un échantillon afin de détecter la présence d'un ou de plusieurs éléments formant analytes à base d'acides nucléiques d'un ensemble d'analytes à base d'acides nucléiques, ledit procédé comprenant, dans n'importe quel ordre adéquat, :
(A) la fourniture d'un support solide ;
(B) l'immobilisation d'au moins deux compositions d'amorce d'ADN distinctes sur une surface du support solide à des emplacements distincts ; dans lequel chacune des compositions d'amorce d'ADN distinctes comprend une amorce d'ADN qui s'hybride dans des conditions stringentes à un élément différent dudit ensemble d'analytes à base d'acides nucléiques ; et
(C) le dépôt d'une polymérase aux mêmes emplacements distincts sur la surface du support solide en utilisant la technologie par jet pulsé.

2. Procédé selon la revendication 1, dans lequel les compositions d'amorce d'ADN comprennent en outre :
(i) dATP ;
(ii) dGTP ;
(iii) dTTP ;
(iv) dUTP ;
(v) dCTP ; ou
(vi) au moins un type de dNTP marqué.

3. Procédé selon la revendication 1 ou 2, dans lequel ladite polymérase comprend une activité qui est une ADN polymérase, une ARN polymérase ou une transcriptase inverse.

4. Procédé selon l'une quelconque des revendications 1 à 3, dans lequel chacune desdites compositions d'amorce comprend en outre un inhibiteur de l'ARNase.

5. Procédé selon l'une quelconque des revendications précédentes, dans lequel l'arrangement de compositions d'amorce d'ADN est sous un format sec et stable lors du stockage, chaque composition d'amorce d'ADN comprenant :
(a) une amorce d'ADN ; et
(b) au moins une quantité efficace d'un réactif de synthèse de l'ADN qui est :
(i) dATP ;
(ii) dGTP ;
(iii) dTTP ;
(iv) dCTP ; ou
(v) au moins un type de dNTP marqué.

6. Arrangement pouvant être obtenu par le procédé selon l'une quelconque des revendications 1 à 5.

7. Procédé pour analyser un échantillon afin de détecter la présence d'un ou de plusieurs éléments formant analytes à base d'acides nucléiques d'un ensemble d'analytes à base d'acides nucléiques, ledit procédé comprenant :
(a) la fourniture d'un arrangement selon la revendication 6 ;
(b) la mise en contact de chacune desdites au moins deux compositions d'amorce d'ADN distinctes dudit arrangement avec ledit échantillon dans des conditions de synthèse de l'ADN suffisantes pour produire des acides nucléiques cibles marqués à des emplacements sur ladite surface où un analyte à base d'acides nucléiques présent dans ledit échantillon s'hybride à une amorce d'ADN pour produire un acide nucléique en double hélice ;
(c) la détection de la présence d'acides nucléiques cibles marqués sur ladite surface de l'arrangement pour obtenir des données d'analyse ; et
(d) l'utilisation desdites données d'analyse pour déterminer la présence d'un ou de plusieurs analytes à base d'acides nucléiques dans ledit échantillon.

8. Procédé selon la revendication 7, dans lequel ledit procédé est un procédé pour déterminer quantitativement la présence desdits un ou plusieurs analytes à base d'acides nucléiques.

9. Procédé selon la revendication 7 ou 8, dans lequel ledit procédé comprend la mise en contact séquentielle de chaque dite composition d'amorce avec un ou plusieurs réactif(s) supplémentaire(s) d'allongement d'amorce matrice-dépendant et dudit échantillon.

10. Procédé selon l'une quelconque des revendications précédentes, dans lequel ladite étape de mise en contact comprend le dépôt d'un volume d'échantillon sur chaque amorce d'ADN dudit arrangement.

11. Procédé selon la revendication 10, dans lequel ledit volume d'échantillon est déposé par dépôt de liquide par jet pulsé.

12. Procédé selon l'une quelconque des revendications précédentes, dans lequel ladite étape de mise en contact comprend la mise en contact de ladite surface dé l'arrangement entier avec ledit échantillon.
